# EUROPEAN PATENT APPLICATION

(11) **EP 3 903 791 A1**
(43) Date of publication of application: **03.11.2021**
(21) Application number: 19905829.8
(22) Date of filing: 27.12.2019
(51) Int. Cl.: A61K 31/7004, A61K 31/7024, A61P 31/14

(54) **ANTI-HUMAN NOROVIRUS AGENT**

(30) Priority: 27.12.2018 JP 2018244213
(71) Applicant: Keio University, Tokyo, 108-8345 (JP); The Kitasato Institute, Tokyo 108-8641 (JP)
(72) Inventor: KATAYAMA Kazuhiko, Tokyo 108-8641 (JP); FUJIMOTO Akira, Tokyo 108-8641 (JP); HAGA Kei, Tokyo 108-8641 (JP); TODAKA Reiko, Tokyo 108-8641 (JP); SATO Toshiro, Tokyo 160-8582 (JP)
(74) Representative: Henkel & Partner mbB
(86) International application number: PCT/JP2019/051496
(87) International publication number: WO 2020/138447

(57) **Abstract**

An object of the present invention is to provide an anti-human norovirus agent which can inhibit infection with and proliferation of human norovirus. The present inventors have found that the object can be attained through provision of an anti-human norovirus agent comprising, as an active ingredient, a fucose analog having an inhibitory action on glycosylation by fucosyltransferase (FUT). Examples of the active ingredient include a fucose analog represented by formula (III) or (IV) or a salt thereof. 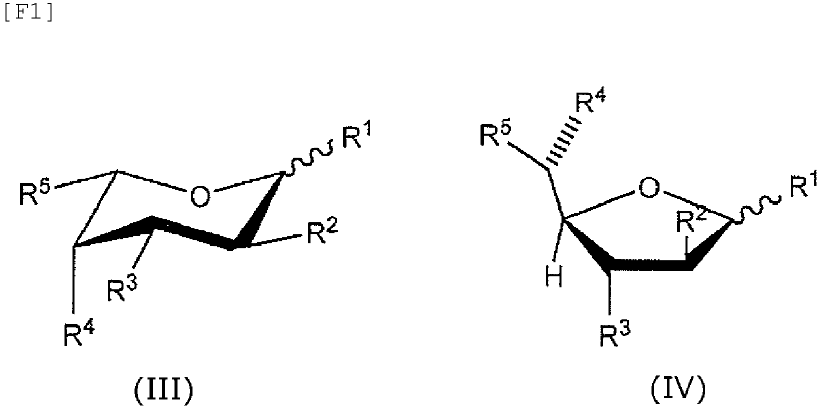 [In the formulas, each of formula (III) and formula (IV) represents an α-anomer or a β-anomer; each of R¹, R³, and R⁴ is -OH or -OAc; R² is a halogen atom, -OH, or -OAc; and R⁵ is -CH₃, -C=CH, -C≡CCH₃, or -CH₂C≡CH.]

## Description

### Technical Field

The present invention relates to a therapeutic agent for infections and, more particularly, to an agent against human norovirus (hereinafter may be referred to as an "anti-human norovirus agent").

### Background Art

Norovirus is one of the four members belonging to the family *Caliciviridae.* Currently, norovirus is a single-species virus, which is Norwalk virus.

Human norovirus infection is caused mainly by the mediation of food (fish and shellfish, or water). The infection may occur even in the presence of a very small number (about 100 to 1,000) of virus particles, indicating considerably strong infection action of human norovirus. Food poisoning caused by human norovirus accounts for more than 90% of all cases of viral food poisoning.

The major symptoms of human norovirus infection include nausea, vomiting, diarrhea, and fever. Infected patients suffer vomiting and diarrhea several times a day, and more than 10 times a day in severe cases. Vomit may contain bile and intestinal substances, which can be extremely distressing.

According to some data of North America, annually, about 20 million persons suffer from human norovirus infection; about 2 million persons visit hospitals; 400,000 persons are conveyed by ambulance; 70,000 persons are hospitalized, and 7,000 persons die.

According to an estimation of the National Institute of Infection of Japan, cases of vomiting/diarrhea caused by human norovirus infection have been increasing to 1 to 3 million people per year over several decades, which has an serious impact on society and the economy. Under such circumstances, Japanese people highly seek an antiviral drug and vaccine against human norovirus, and development thereof has been placed at the fourth administrative position in order of priority. In Japan, there are very few cases where human norovirus infection is a direct cause of death, but a high risk of prolongation and severity is imposed in the case of co-presence of other diseases and in elderly people. Also, even if the aforementioned symptoms are mitigated, excretion of highly contagious human norovirus into feces lasts for 7 to 14 days, or for 2 months or longer in the case of a long period, making the patients unable to return to schools or workplaces during the period. Further, latent human norovirus infection cases are usually detected at a rate of several percent of the total population. Since such a latent human norovirus infection patient is not aware of viral infection by himself or herself, large-scale food poisoning has been reported to be caused by, for example, a latent infection patient working as a food service worker.

As described above, human norovirus is a food-toxic virus which results in high morbidity and which causes pain in patients. In addition, human norovirus is a highly contagious virus causing latent infection. However, no specific medicine against norovirus has hitherto been developed. Basically, oral water supply, drip infusion, or the like is performed for preventing dehydration, with follow-up observation. In addition, an antiemetic agent, an intestinal regulator, or the like is administered as symptomatic therapy. Use of an antidiarrheal is negatively guided at least in an initial onset stage, since norovirus remains in the intestinal tract, thereby retarding recovery.

In 2002, a French study group reported that the virus-like (hollow) particle (VLP) of human norovirus binds to human histo-blood group antigen (HBGA), and suggested that this molecule may be a possible receptor of human norovirus (Non-Patent Document 1).

Thereafter, through progress in verification by human volunteers, it has been elucidated that HBGA non-secretory individuals are less susceptible to human norovirus, and HBGA secretory individuals are more susceptible to human norovirus (Non-Patent Document 2).

However, at present, HBGA *per se* is not regarded as a receptor responsible for the invasion of human norovirus into cells, for the following reasons.

The status of a secretor (i.e., secretor status); that is, whether or not HBGA is secreted into saliva and mucous membrane, is thought to be related to the genetic polymorphism of FUT2 (fucosyltransferase 2 of fucosyltransferases 1-8). However, even if the FUT2 gene is introduced into a cell in which HBGA is not formed through gene expression on the cell surface so as to secrete HBGA and form HBGA through gene expression on the cell surface, infection of the cell with human norovirus is not established. Thus, HBGA itself is not regarded as a receptor responsible for invasion of human norovirus into cells, and the causal relationship (directly and indirectly) between the FUT2 gene polymorphism and human norovirus infection susceptibility has not been elucidated (Non-Patent Document 3).

Patent Document 1 is a prior art document which discloses use, as an anti-cancer agent, of the fucose analog serving as the active ingredient of the below-mentioned anti-norovirus agent of the present invention. Although Patent Document 1 generally discloses use of the fucose analog for the treatment of infections, no specific working examples thereof are disclosed. The viruses listed in Patent Document 1 do not include norovirus and related diarrhea viruses. Furthermore, the action mechanism against infections disclosed in Patent Document 1 is an enhanced immuno-response, which completely differs from that of the anti-norovirus agent of the present invention.

Patent Document 2 discloses a 2D organoid used in the Examples.

### Prior Art Documents

### Patent Documents

Patent Document 1: Japanese Patent No. 5918235
Patent Document 2: WO 2018/038042, pamphlet
Patent Document 3: Japanese Patent Application Laid-Open (kokai) No. 2018-2666

### Non-Patent Documents

Non-Patent Document 1: Marionneau, S. *et al.,*
GASTROENTEROLOGY 122: 1967-1977(2002)
Non-Patent Document 2: Hutson, A. M. et al., Journal of Medical Virology 77: 116-120(2005)
Non-Patent Document 3: Guix, S. et al., J. Virol., 81(22): 12238-12248(2007)

### Summary of the Invention

### Problems to be Solved by the Invention

An object of the present invention is to provide an anti-norovirus agent having a substantial therapeutic effect to human norovirus.

### Means for Solving the Problems

The present inventors have conducted extensive studies for solving the aforementioned problems, and have surprisingly found that human norovirus infection of intestinal cells, which are susceptible to infection with human norovirus, and further, proliferation of the human norovirus in the intestinal cells, can be suppressed through administration of a fucose analog which inhibits glycosylation by fucosyltransferase (FUT). The present invention has been accomplished on the basis of this finding.

Accordingly, the present invention provides an anti-human norovirus agent comprising, as an active ingredient, a fucose analog which inhibits glycosylation by fucosyltransferase (FUT), or a salt thereof (hereinafter the agent may be referred to as "the anti-norovirus agent of the present invention"). Preferably, the fucosyltransferase includes FUT2.

The present invention will be described in more detail.

The anti-norovirus agent of the present invention is an anti-human norovirus agent comprising, as an active ingredient, a fucose analog represented by the below-provided formula (I) or (II) or a salt thereof (hereinafter, unless otherwise specified, the term "fucose analog" also encompasses a salt thereof). Notably, in the present description, a functional group may be denoted by an element symbol and "-". For example, "-OH" denotes a hydroxyl group. The number of carbon atoms may be represented as, for example, C₁-C₁₀ (i.e., the number of carbon atoms being 1 to 10). The term "salt" refers to a pharmaceutically or biologically acceptable salt and may be any of a neutral salt, an acidic salt, and a basic salt. 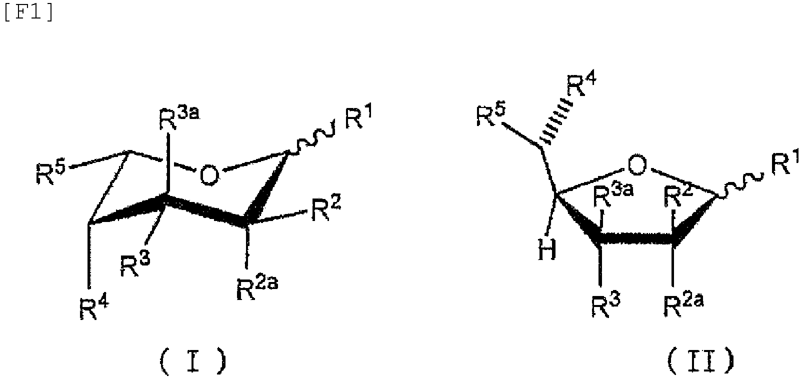 [In the above formulas, each of formulas (I) and (II) represents an **α-**anomer or a **β-**anomer;
each of R¹, R², R³, and R⁴ is selected from the group consisting of a fluorine atom (F), a chlorine atom (Cl), -OH, -OC(O)H, -OC(O)C₁-C₁₀ alkyl, -OC(O)C₂-C₁₀ alkenyl, -OC(O)C₂-C₁₀ alkynyl, -OC(O)aryl, -OC(O)heterocycle, -OC(O)C₁-C₁₀ alkylene (aryl), -OC(O)C₂-C₁₀ alkenylene (aryl), -OC(O)C₂-C₁₀ alkynylene(aryl), -OC(O)C₁-C₁₀ alkylene(heterocycle), - OC(O)C₂-C₁₀ alkenylene (heterocycle) , -OC(O)C₂-C₁₀ alkynylene(heterocycle), -OCH₂OC(O)alkyl, -OCH₂OC(O)O-alkyl-OCH₂OC(O) aryl, -OCH₂OC(O)O-aryl, -OC(O)CH₂O(CH₂CH₂O)ₙCH₃,-OC(O)CH₂CH₂O(CH₂CH₂O)ₙCH₃, -O-tri-C₁-C₃ alkylsilyl, and -OC₁-C₁₀ alkyl. Each n is an integer of 0 to 5;
each of R^{2a} and R^{3a} is selected from the group consisting of a hydrogen atom (H), F, and Cl;
R⁵ is selected from the group consisting of -CH₃, -CHF₂, -CH=C=CH₂, -C=CH, -C≡CCH₃, -CH₂C≡CH, -C(O)OCH₃, -CH(OAc)CH₃,-CN, -CH₂CN, -CH₂X (X represents F, a bromine atom (Br), Cl, or an iodine atom (I)), and methoxysilane; and
when R⁵ is a group other than -CH=C=CH₂, -CH₂F, or -CHF₂, at least one of R¹, R², R³, R^{2a}, and R^{3a} is F or Cl.]

Among the above-mentioned members, the fucose analog or a salt thereof, which serves as an active ingredient of the anti-norovirus agent of the present invention, is preferably a fucose analog represented by formula (I) or a salt thereof. In addition to the salt form, a form of solvate is also acceptable.

The fucose analog represented by formula (I) or (II) is known to exhibit a fucosylation reducing effect of at least 10%, with respect to fucosylation of a protein in the case where no fucose analog is administered (Patent Document 1). In the present invention, "inhibition of glycosylation by fucosyltransferase (FUT)" means a fucosylation reduction effect of 10% or more.

The norovirus targeted by the anti-norovirus agent of the present invention is human norovirus. The anti-norovirus agent of the present invention can suppress human norovirus infection of intestinal cells, which are susceptible to infection with human norovirus, and proliferation of the human norovirus in the intestinal cells.

The fucose analog, which serves as an active ingredient of the anti-norovirus agent of the present invention, preferably has cell membrane permeability to human cells, at least to human intestinal cells, for exerting the anti-norovirus effect thereof. So long as the FUT inhibition effect of the fucose analog is not impaired, it is preferable that one or more groups selected from R¹, R², R³, R^{2a}, and R^{3a} are "-OC(O)C₁-C₁₀ alkyl," preferably O-acetyl(OAc); i.e., "-OC(O)CH₃" (Patent Document 3).

The fucose analog, which serves as an active ingredient of the anti-norovirus agent of the present invention, inhibits glycosylation by fucosyltransferase (FUT) in intestinal cells. Particularly, inhibition of glycosylation by FUT2 is observed along with the anti-norovirus effect.

The fucose analog represented by the aforementioned formula (I) or (II) is preferably a fucose analog represented by the below-provided formula (III) or (IV) or a salt thereof. 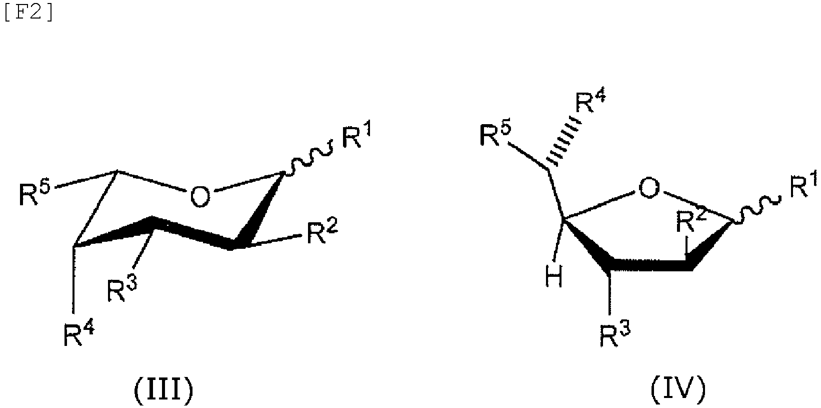 [In the above formulas, each of formulas (III) and (IV) represents an α-anomer or a β-anomer;
each of R¹, R³, and R⁴ is -OH or -OAc;
R² is a halogen atom which is F (fluorine atom) or Cl (chlorine atom), -OH, or -OAc; and
R⁵ is -CH₃, -C=CH, -C≡CCH₃, or -CH₂C≡CH.]

(1) In the above formula (III) or (IV), when R² is a halogen atom which is F (fluorine atom) or Cl (chlorine atom), at least one of R¹, R³, and R⁴ is preferably -OAc. More preferably, two or more groups of R¹, R³, and R⁴ are -OAc. Most preferably, all of R¹, R³, and R⁴ are -OAc.

In the above formula (III) or (IV), when R² is a halogen atom which is F (fluorine atom) or Cl (chlorine atom), R⁵ is preferably -CH₃, and the halogen atom is preferably F (fluorine atom).

(2) In the above formula (III) or (IV), when R⁵ is -C=CH, -C≡CCH₃, or -CH₂C≡CH, R² is preferably -OH or -OAc. At least one group of R¹, R², R³, and R⁴ is preferably -OAc. More preferably, two or more groups of R¹, R², R³, and R⁴ are -OAc. Most preferably, all of R¹, R², R³, and R⁴ are -OAc. In addition, in a preferred embodiment, the alkynyl group is - C=CH.

(3) In (1) and (2) above, the fucose analog or a salt thereof, which serves as an active ingredient of the anti-norovirus agent of the present invention, is preferably a fucose analog represented by formula (III) or a salt thereof. In addition to the salt form, a form of solvate is also acceptable.

Conceivably, when the fucose analog represented by formula (I) or (II), or formula (III) or (IV), or a salt or solvate thereof, which serves as an active ingredient of the anti-norovirus agent of the present invention, is incorporated into intestinal cells, the fucose analog replaces the fucose moiety of GDP-fucose (i.e., a metabolic intermediate), to thereby inhibit normal glycosylation and, via this action, provide an anti-norovirus effect of interest. However, no precise mechanism thereof has been elucidated. More specifically, (a) invasion of human norovirus into intestinal cells (i.e., infection of the intestinal cells with human norovirus) is inhibited via the aforementioned action. However, as described above, there has not been found a receptor responsible for the invasion of human norovirus present on the cell surface into the cells. As has been recognized in relation to HIV, HCV, and HBV, a plurality of receptor molecules might be concertedly involved with infection of human norovirus. Thus, characterization of the receptor is currently in a chaotic state. In contrast, surprisingly, the fucose analog serving as an active ingredient of anti-norovirus agent of the present invention acts on an unknown receptor molecule or receptor molecule groups (one or more species thereof), whereby infection of intestinal cells with human norovirus is inhibited. More surprisingly, (b) with the above action, proliferation of human norovirus itself in intestinal cells can be inhibited.

### Effects of the Invention

The present invention enables provision of an anti-norovirus agent which inhibits infection of intestinal cells with human norovirus and proliferation of the human norovirus in the intestinal cells and which can prevent and treat human norovirus infections.

### Brief Description of the Drawings

[FIG. 1] A chart showing lineages of norovirus, in particular three lineages (Lineages 1 to 3) to which norovirus GII (frequent genotype of human norovirus) belongs.
[FIG. 2] A graph showing the results of studies on cytotoxicity of 2fp-fucose to a 2D organoid derived from intestinal stem cells.
[FIG. 3] Photographs showing the results of studies on (a) reduction of HBGA and (b) reduction of VLP adsorption, on the surface of a 2D organoid derived from intestinal stem cells, through treatment with 2fp-fucose.
[FIG. 4] Graphs showing the effect of 2fp-fucose treatment on isolated strains of norovirus GII lineages 1 to 3 in a 2D organoid derived from intestinal stem cells.
[FIG. 5] Graphs showing the effect of 2fp-fucose treatment (Days 1 to 4) on proliferation of human norovirus in a 2D organoid derived from intestinal stem cells.
[FIG. 6] A graph showing comparison of 2fp-fucose with other viral proliferation inhibitors, in terms of human norovirus proliferation inhibiting effect in a 2D organoid derived from intestinal stem cells.
[FIG. 7] A graph showing the RT-PCR results of studies on FUT (from FUT1 to FUT11) formation via gene expression in a 2D organoid derived from intestinal stem cells.
[FIG. 8] Western blotting results of studies on FUT2 gene expression in an FUT2 gene-knocked-out 2D organoid derived from intestinal stem cells and the wild-type 2D organoid equivalent.
[FIG. 9] Photographs showing the state of HBGA on cell surface in an FUT2 gene-knocked-out 2D organoid derived from intestinal stem cells and the wild-type 2D organoid equivalent, on the basis of binding of FITC (phosphor)-labeled UEA-1 as an index.
[FIG. 10] Photographs showing the results of a VLP binding test to an FUT2 gene-knocked-out 2D organoid derived from intestinal stem cells and the wild-type 2D organoid equivalent.
[FIG. 11] A photograph showing the results of confirmation of binding of VLP to an FUT2 gene-knocked-out 2D organoid derived from intestinal stem cells, after re-incorporation of the FUT gene into the organoid.
[FIG. 12] A graph showing a comparative study to determine percent proliferation in each of a wild-type 2D organoid derived from intestinal stem cells, an FUT2 gene-knocked-out 2D organoid equivalent, and an FUT2 gene-reincorporated 2D organoid equivalent, after infection with human norovirus.
[FIG. 13] Graphs showing the test results of norovirus proliferation inhibition by 6a-fucose.
[FIG. 14] Graphs showing the effects of 2fp-fucose treatment and 6a-fucose treatment, after viral infection.

### Modes for Carrying Out the Invention

### [1] Fucose analogs

As mentioned above, the active ingredient of the anti-norovirus agent of the present invention is a fucose analog (I) or (II) [including (III) or (IV)], a salt thereof, or, in some cases, a solvate thereof.

In each of R¹, R², R³, and R⁴ which can independently represent an atom or a functional group, the "alkyl group" is a substituted or unsubstituted, saturated linear-chain or saturated branched hydrocarbyl group having a predetermined number of carbon atoms.

Examples of the unsubstituted alkyl group include methyl, ethyl, n-propyl, isopropyl, n-butyl, isobutyl, sec-butyl, tert-butyl, n-pentyl, 1-methylbutyl, 2-methylbutyl, 3-methylbutyl, 1,1-dimethylpropyl, 1,2-dimethylpropyl, 2,2-dimethylpropyl, n-hexyl, 2-hexyl, 3-hexyl, n-heptyl, n-octyl, n-nonyl, n-decyl, 1-methylbenzyl, 2-methylbenzyl, 3-methylbenzyl, 4-methylbenzyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl.

The substituted alkyl group may be an alkyl group substituted by a halogen atom, or may be an alkyl group substituted by one or more groups, preferably 1 to 3 groups, selected from among -O-(C₁-C₈ alkyl), -O-(C₂-C₈ alkenyl), -O-(C₂-C₈ alkynyl), aryl, -C(O)R', -OC(O)R', -C(O)OR', -C(O)NH₂, -C(O)NHR', -C(O)N(R')₂, -NHC(O)R', -SR', -SO₃R', -S(O)₂R', - S(O)R', -OH, =O, -NH₂, -NH(R'), -N(R')₂, -CN, etc. R' is independently selected from -H, -C₁-C₈ alkyl, -C₂-C₈ alkenyl, -C₂-C₈ alkynyl, and aryl.

Examples of possible R' of the alkenyl group include, but are not limited to, ethenyl, vinyl, allyl, 1-butenyl, 2-butenyl, isobutylenyl, 1-pentenyl, 2-pentenyl, 3-methyl-1-butenyl, 2-methyl-2-butenyl, and 2,3-dimethyl-2-butenyl.

Examples of possible R' of the alkynyl group include, but are not limited to, ethynyl, propynyl, 1-butynyl, 2-butynyl, 1-pentynyl, 2-pentynyl, and 3-methyl-1-pentynyl.

In each of R¹, R², R³, and R⁴ which can independently represent an atom or a functional group, each of the "alkenyl group" and the "alkynyl group" is a substituted or unsubstituted, unsaturated linear-chain or unsaturated branched hydrocarbyl group having a predetermined number of carbon atoms.

The alkenyl chain has at least one double bond in the chain, and the alkynyl chain has at least one triple bond in the chain.

Similar to the examples of possible R' of the alkenyl group, examples of the unsubstituted alkenyl group include, but are not limited to, ethenyl, vinyl, allyl, 1-butenyl, 2-butenyl, isobutylenyl, 1-pentenyl, 2-pentenyl, 3-methyl-1-butenyl, 2-methyl-2-butenyl, and 2,3-dimethyl-2-butenyl.

Similar to the examples of possible R' of the alkynyl group, examples of the unsubstituted alkynyl group include, but are not limited to, ethynyl, propynyl, 1-butynyl, 2-butynyl, 1-pentynyl, 2-pentynyl, and 3-methyl-1-pentynyl.

When being substituted, the alkenyl group and the alkynyl group may be substituted by a halogen atom, or may be substituted by one or more groups, preferably 1 to 3 groups, selected from among -O-(C₁-C₈ alkyl), -O-(C₂-C₈ alkenyl), -O-(C₂-C₈ alkynyl), aryl, -C(O)R', -OC(O)R', -C(O)OR', -C(O)NH₂, -C(O)NHR', -C(O)N(R')₂, -NHC(O)R', -SR', -SO₃R', -S(O)₂R', - S(O)R', -OH, =O, -NH₂, -NH(R'), -N(R')₂, -CN, etc. Definition of R' is the same as mentioned above.

In each of R¹, R², R³, and R⁴ which can independently represent an atom or a functional group, the "alkylene (group)" denotes a substituted or unsubstituted, saturated branched or saturated linear-chain hydrocarbyl group including two monovalent core moieties and having a predetermined number of carbon atoms. Typical examples of the unsubstituted alkylene group include, but are not limited to, methylene, ethylene, propylene, butylene, pentylene, hexylene, heptylene, octylene, nonylene, decylene, and 1,4-cyclohexylene.

The substituted alkylene group may be an alkylene group substituted by a halogen atom, or may be an alkylene group substituted by one or more groups, preferably 1 to 3 groups, selected from among -O-(C₁-C₈ alkyl), -O-(C₂-C₈ alkenyl), -O-(C₂-C₈ alkynyl), aryl, -C(O)R', -OC(O)R', -C(O)OR', -C(O)NH₂, -C(O)NHR', -C(O)N(R')₂, -NHC(O)R', -SR', -SO₃R', -S(O)₂R', - S(O)R', -OH, =O, -NH₂, -NH(R'), -N(R')₂, -CN, etc. Definition of R' is the same as mentioned above.

In each of R¹, R², R³, and R⁴ which can independently represent an atom or a functional group, the "alkenylene (group)" denotes an unsaturated branched, linear-chain, or cyclic hydrocarbyl group including two monovalent core moieties derived through elimination of two hydrogen atoms from a single or two different carbon atoms of the parent alkene of the aforementioned alkenyl group. The "alkenylene" group may be unsubstituted, or may be substituted as in the case of the alkenyl group. In some embodiments, the "alkenylene" group is unsubstituted.

The "alkynylene (group)" is a substituted or unsubstituted, unsaturated branched, unsaturated linear-chain, or unsaturated cyclic hydrocarbyl group including two monovalent core moieties derived through elimination of two hydrogen atoms from a single or two different carbon atoms of the parent alkyne of the aforementioned alkynyl group.

In each of R¹, R², R³, and R⁴ which can independently represent an atom or a functional group, the "aryl (group)" refers to a substituted or unsubstituted monovalent aromatic hydrocarbyl group having 6 to 20 carbon atoms, which group is derived through elimination of one hydrogen atom from one carbon atom of the parent aromatic ring system. Some aryl groups are represented by a symbol "Ar" in an exemplary structure. Typical examples of the unsubstituted aryl group include, but are not limited to, groups derived from benzene, substituted benzene, phenyl, naphthalene, anthracene, biphenyl, etc.

The substituted aryl group may be an aryl group substituted by a halogen atom, or may be an aryl group substituted by one or more groups, preferably 1 to 3 groups, selected from among -O-(C₁-C₈ alkyl), -O-(C₂-C₈ alkenyl), -O-(C₂-C₈ alkynyl), aryl, -C(O)R', -OC(O)R', -C(O)OR', -C(O)NH₂, -C(O)NHR', -C(O)N(R')₂, -NHC(O)R', -SR', -SO₃R', -S(O)₂R', - S(O)R', -OH, =O, -NH₂, -NH(R'), -N(R')₂, -CN, etc. Definition of R' is the same as mentioned above

In each of R¹, R², R³, and R⁴ which can independently represent an atom or a functional group, the "heterocycle" refers to a substituted or unsubstituted single cyclic system having 3 to 7 or 3 to 10 ring-forming atoms in which at least one of them is a hetero atom selected from N, O, P, and S. One or more N, C, or S atoms in the heterocycle may be oxidized. A single heterocycle preferably has a 3- to 7-membered ring (e.g., 2 to 6 carbon atoms and 1 to 3 hetero atoms independently selected from N, O, P, and S). The hetero atom-containing ring may be an aromatic ring or a nonaromatic ring. Unless otherwise specified, the heterocycle is bound at any hetero atom or any carbon atom thereof that provides structural stability.

The definition of "heterocycle" is described in Paquette, "Principles of Heterocyclic Chemistry" (W.A. Benjamin, New York, 1968), particularly in Chapters 1, 3, 4, 6, 7, and 9; "The Chemistry of Heterocyclic Compounds, A Series of Monographs" (John Wiley & Sons, New York, 1950 to), particularly in Chapters 13, 14, 16, 19, and 28; and J. Am. Chem. Soc., 82:5566 (1960). Examples of the unsubstituted "heterocycle" group include, but are not limited to, pyridyl, dihydropyridyl, tetrahydropyridyl (piperidyl), thiazolyl, pyrimidinyl, furanyl, thienyl, pyrrolyl, pyrazolyl, imidazolyl, tetrazolyl, fucosyl, aziridinyl, azetidinyl, oxyranyl, oxetanyl, and tetrahydrofuranyl.

The substituted heterocycle group may be a heterocycle group substituted by a halogen atom, or may be a heterocycle group substituted by one or more groups, preferably 1 to 3 groups, selected from among -O-(C₁-C₈ alkyl), -O-(C₂-C₈ alkenyl), -O-(C₂-C₈ alkynyl), aryl, -C(O)R', -OC(O)R', - C(O)OR', -C(O)NH₂, -C(O)NHR', -C(O)N(R')₂, -NHC(O)R', -SR', - SO₃R', -S(O)₂R', -S(O)R', -OH, =O, -NH₂, -NH(R'), -N(R')₂, -CN, etc. Definition of R' is the same as mentioned above

More specific examples of the heterocycle group include a carbon-bound heterocycle group. The positions of the heterocycle available for bonding at a carbon atom are as follows: 2-, 3-, 4-, 5-, or 6-position of pyridine; 3-, 4-, 5-, or 6-position of pyridazine; 2-, 4-, 5-, or 6-position of pyrimidine; 2-, 3-, 5-, or 6-position of pyrazine; 2-, 3-, 4-, or 5-position of furan, tetrahydrofuran, thiofuran, thiophene, pyrrole, or tetrahydropyrrole; 2-, 4-, or 5-position of oxazole, imidazole, or thiazole; 3-, 4-, or 5-position of isoxazole, pyrazole, or isothiazole; 2- or 3-position of aziridene; and 2-, 3-, or 4-position of azetidine.

Examples of the carbon-bound heterocycle group include 2-pyridyl, 3-pyridyl, 4-pyridyl, 5-pyridyl, 6-pyridyl, 3-pyridazinyl, 4-pyridazinyl, 5-pyridazinyl, 6-pyridazinyl, 2-pyrimidinyl, 4-pyrimidinyl, 5-pyrimidinyl, 6-pyrimidinyl, 2-pyridazinyl, 3-pyridazinyl, 5-pyridazinyl, 6-pyridazinyl, 2-thiazolyl, 4-thiazolyl, or 5-thiazolyl.

The nitrogen-bound heterocycle group may be bound at 1-position of aziridine, azetidine, pyrrole, pyrrolidine, 2-pyrroline, 3-pyrroline, imidazole, imidazolidine, 2-imidazoline, 3-imidazoline, pyrazole, pyrazoline, 2-pyrazoline, 3-pyrazoline, piperidine, piperazine, indole, indoline, or 1H-indazole; 2-position of isoindole or isoindoline; and 4-position of morpholine. Typical examples of the nitrogen-bound heterocycle group include 1-aziridyl, 1-azetidyl, 1-pyrrolyl, 1-imidazolyl, 1-pyrazolyl, and 1-piperidinyl.

The "C₁-C₁₀ alkyl" of "-OC(O)C₁-C₁₀ alkyl," which R¹, R³, and R⁴ can independently represent, is an alkyl group having 1 to 10 carbon atoms and a substituted or unsubstituted, saturated linear-chain or saturated branched hydrocarbyl group having the corresponding number of carbon atoms. Examples of the unsubstituted alkyl group include methyl, ethyl, n-propyl, isopropyl, n-butyl, isobutyl, sec-butyl, tert-butyl, n-pentyl, 1-methylbutyl, 2-methylbutyl, 3-methylbutyl, 1,1-dimethylpropyl, 1,2-dimethylpropyl, 2,2-dimethylpropyl, n-hexyl, 2-hexyl, 3-hexyl, n-heptyl, n-octyl, n-nonyl, n-decyl, 1-methylbenzyl, 2-methylbenzyl, 3-methylbenzyl, 4-methylbenzyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl, and 1-ethyl-2-methylpropyl. When the unsubstituted alkyl group is a methyl group, the "-OC(O)C₁-C₁₀ alkyl" is -O-acetyl(-OAc); i.e., "-OC(O)CH₃," which is exemplified as a preferable embodiment of "-OC(O)C₁-C₁₀ alkyl." In this case, in one preferred embodiment, one or more groups of R¹, R³, and R⁴ are -OAc. In one more preferred embodiment, two or more groups of R¹, R³, and R⁴ are -OAc. In one most preferred embodiment, all three groups of R¹, R³, and R⁴ are -OAc.

In addition, the substituted alkyl group may be an alkyl group substituted by a halogen atom, or may be an alkyl group substituted by one or more groups, preferably 1 to 3 groups, selected from among -O-(C₁-C₈ alkyl), -O-(C₂-C₈ alkenyl), -O-(C₂-C₈ alkynyl), aryl, -C(O)R', -OC(O)R', - C(O)OR', -C(O)NH₂, -C(O)NHR', -C(O)N(R')₂, -NHC(O)R', -SR', - SO₃R', -S(O)₂R', -S(O)R', -OH, =O, -NH₂, -NH(R'), -N(R')₂, -CN, etc. R' is independently selected from among -H, -C₁-C₈ alkyl, -C₂-C₈ alkenyl, -C₂-C₈ alkynyl, and aryl.

Examples of possible R' of the alkenyl group include, but are not limited to, ethenyl, vinyl, allyl, 1-butenyl, 2-butenyl, isobutylenyl, 1-pentenyl, 2-pentenyl, 3-methyl-1-butenyl, 2-methyl-2-butenyl, and 2,3-dimethyl-2-butenyl.

Examples of possible R' of the alkynyl group include, but are not limited to, ethynyl, propynyl, 1-butynyl, 2-butynyl, 1-pentynyl, 2-pentynyl, and 3-methyl-1-pentynyl.

The fucose analog (I) or (II) [including (III) or (IV)], a salt thereof, or a solvate thereof may be produced through a known method. For example, as disclosed in Patent Document 3, L-galactose is used as a starting material, and the corresponding hydroxyl groups thereof are protected via formation of an isopropylidene moiety. The side chains other than the protected moiety are transformed into a substituent of interest (e.g., addition of an alkynyl group to 5-position of carbon atom) by known means. The isopropylidene moiety is removed for deprotection, and the deprotected hydroxyl groups may optionally be subjected to a known acetylation treatment.

The fucose analog used in the invention may be a commercial product (including outsourcing synthesis).

### [2] Pharmaceutical composition

The fucose analog (I) or (II) [including (III) or (IV)], a salt thereof, or a solvate thereof (in the section of "Pharmaceutical composition," collectively also referred to as a "fucose analog") is an active ingredient of the anti-norovirus agent of the present invention. The fucose analog as is can be used as an anti-norovirus agent. Alternatively, the fucose analog may be appropriately formulated, to thereby provide a pharmaceutical composition (hereinafter may also be referred to simply as a "composition"). The composition contains a therapeutically effective amount of the fucose analog and, typically, one or more carriers (e.g., sterilized liquids such as water or oil, including an oily ingredient synthesized or derived from petroleum, animal, or plant, for example, peanut oil, soybean oil, mineral oil, or sesame oil). Water is a typical carrier of the composition for intravenous administration. Further, physiological saline and, water solution of dextrose and glycerol may also be used as a liquid carrier for injection. Examples of appropriate excipients include amino acid, starch, glucose, lactose, sucrose, gelatin, malt, rice, wheat flour, chalk, silica gel, sodium stearate, glyceryl monostearate, talc, sodium chloride, dry skim milk, glycerol, propylene glycol, water, and ethanol. If needed, the composition may optionally contain a hygro-spreader, an emulsifier, a pH buffer, a disintegrant, a sweetener, a preservative, a dye/colorant, etc. The thus-provided compositions may be in the form of solution, syrup, elixir, suspension, enteric agent, emulsion, tablet, pill, capsule, powder, sustained release preparation, or the like.

When the composition has a capsule form such as an enteric capsule or gelatin capsule, the composition may further contain a liquid carrier such as polyethylene glycol, cyclodextrin, or fatty oil, in addition to the aforementioned materials.

The composition may have a fucose analog content falling within a range of 0.01 to 99 mass%, depending on dosage form, mode of administration, etc.

In administration of the anti-norovirus agent of the present invention to human subjects, the dosage of the fucose analog is 0.01 to 500 mg/kg-body weight, more preferably 0.1 to 200 mg/kg-body weight.

The mode of administering the anti-norovirus agent of the present invention may be chosen according to the purpose.

For example, the anti-norovirus agent of the present invention is administered to a subject in order to prevent infection of the subject with human norovirus, before eating a food (e.g., shellfish) potentially and latently infected with human norovirus in the period of prevalence of human noroviral gastroenteritis, or before traveling to an area where human noroviral infection is prevalent. In such a case, administration of the anti-norovirus agent is started at least four days before the day of eating or traveling, preferably 3 to 4 days before the day. The administration interval should be determined so that the fucose analog concentration of the body does not considerably lower over time during the administration period. The interval varies depending on the form and amount of dosage, but the agent is normally administered at least once a day.

In the case where a subject is affected by human noroviral gastroenteritis, the agent is administered as soon as possible. As described above, the administration interval should be determined so that the fucose analog concentration of the body does not considerably lower over time during the administration period. The interval varies depending on the form and amount of dosage, but the agent is normally administered at least once a day. The administration is preferably continued until the human noroviral RNA disappears in the feces of the subject.

As mentioned hereinbelow, the anti-norovirus agent of the present invention prevents human norovirus infection in intestinal cells. After termination of the administration, the fucose derivative is exhausted from the intestinal cells, providing no cytotoxicity. Thus, the agent of the present invention is milder to the human body. By virtue of the above characteristics, the agent of the present invention can be easily administered for a long period of time. As a result, the anti-norovirus agent of the present invention can be positively administered to a human subject having a reduced tolerance or in immunodeficiency, or to elderly person. In addition, since the anti-norovirus agent of the present invention can be administered for a long period, secondary infections can be avoided by the administration thereof after disappearance of the symptoms of poisoning. Examples

A working example of the present invention will next be described. However, the example should not be construed as limiting the scope of the present invention thereto. In the section of "Examples," the active ingredient of the anti-norovirus agent of the present invention may also be referred to as a "fucose analog."

### [Materials]

### (1) 2D organoid derived from intestinal stem cells and culturing of human norovirus

The 2D organoid derived from intestinal stem cells refers to a 2D organoid for infection and culture of human diarrhea virus (including norovirus) disclosed in Patent Document 2. According to Patent Document 2, the 2D organoid is "obtained by Step 1 in which human intestinal epithelial stem cells, human intestinal epithelial cells, or a tissue including at least one of these cells is subjected to 3-dimensional culturing on the extracellular matrix, to thereby obtain a 3D organoid; and

by Step 2 in which the above-obtained 3D organoid is dispersed to prepare single cells; the single cells are subjected to monolayer culturing on an extracellular matrix; and there is obtained a 2D organoid having a single-layer structure of the epithelial cells for forming the human intestinal cavity which include differentiated trophoblastic cells and caliciform cells." By virtue of the 2D organoid, culturing human norovirus, which was not previously achieved, can be successfully performed.

In the Example, a 2D organoid derived from norovirus-susceptible human intestinal stem cells was prepared through a technique disclosed in paragraphs [0130] to [0139] of Patent Document 2, and used in the tests carried out in the Example.

Specific steps thereof will next be described. The culture media and agents employed in these steps may be selected from corresponding commercial products, or may be produced in the laboratory in accordance with needs.

### (a) Cell culture media

To an Advanced DMEM/F-12 medium (product of, for example, Thermo Fisher SCIENTIFIC), human recombinant R-Spondin 1 (product of, for example, R&D systems) is added to a final concentration of 1 µg/mL; Noggin (product of, for example, Peprotech) is added to a final concentration of 100 ng/mL; and A83-01 (product of, for example, Tocris) is added to a final concentration of 500 nM. The thus-prepared medium is referred to as an "NRA medium."

Subsequently, any of Wnt3a (final concentration: 300 ng/mL), IGF1 (product of, for example, Biolegend) (final concentration: 500 ng/mL), FGF2 (product of, for example, Peprotech) (final concentration: 50 ng/mL), and EGF (product of, for example, Thermo Fisher SCIENTIFIC) (final concentration: 50 ng/mL), are added thereto, to thereby prepare the following combination media.
- WNRA + IGF1 + FGF2 medium
- ENRA medium

Definitions of the abbreviations of the media are as follows.
W: Wnt3A
E: EGF
N: Noggin
R: R-Spondin 1
A: A83-01

### (b) Preparation of 2D organoid

In one procedure, according to the research plan approved by Keio University School of Medicine, the Ethics Committee, normal mucous membrane samples (a portion **≥**5 cm away from the tumor) are collected from healthy subjects and gastrointestinal tract tumor patients from which informed consent has been obtained. Each collected tissue sample is treated with EDTA or Liberase TH, to thereby extract epithelial cells. The cells are embedded in Matrigel (registered trademark).

Epithelial cells (hereinafter may be referred to as intestinal stem cells) embedded in Matrigel (registered trademark) are disseminated onto a 48-well plate and cultured. The specific procedure conducted is as follows.

Cultured intestinal stem cells were disseminated with 25 **µ**L of Matrigel (registered trademark) (product of BD Bioscience) to a 48-well plate. The WNRA + IGF1 + FGF2 medium prepared in (a) was added to each well (100 **µ**L/well), and the cells were cultured at 37°C. From the start of culturing, the medium was exchanged every two days. The culturing was continued for 7 days, to thereby yield a 3D organoid derived from the intestinal stem cells.

Separately, 2.5% Matrigel (registered trademark) diluted with PBS(-) was added to a 96-well plate (50 **µ**L/well), and the plate was subjected to incubation at 37°C for 1 hour or longer. Subsequently, the wells were washed thrice with PBS(-), to thereby produce a well plate for preparing a 2D organoid.

The aforementioned 3D organoid is degraded by use of, for example, TrypLE (trademark) Express (product of Thermo Fisher SCIENTIFIC), to thereby yield single cells. The thus-obtained single cells are washed with an ENRA medium and suspended in an ENRA medium containing Y-27632 (Rock inhibitor, product of, for example, Wako Pure Chemical) (final concentration: 10 **µ**M). The cell suspension is disseminated onto the wells of the aforementioned well plate at 1 × 10⁵ cells/well, and allowed to stand for 3 hours or longer while the cells remain in contact with the plate, to thereby produce a 2D organoid derived from the intestinal stem cells. Unless otherwise specified, the "2D organoid derived from the intestinal stem cells" will be described simply as a "2D organoid."

### (c) Culturing of human norovirus

Feces of a human norovirus-infected patent contains a large amount of infectious human norovirus. Thus, the feces of a human norovirus-infected patient is suspended in distilled water or the like to a final concentration of about 10%, to thereby prepare a "10% feces suspension." The 10% feces suspension is about 10-fold diluted with an ENRA medium. The diluted suspension is added to each of the aforementioned wells having the 2D organoid (5 **µ**L/well) and subjected to incubation for about 3 hours, to thereby proliferate human norovirus. As a result of verification, it has been confirmed that human norovirus proliferates to about 100,000 times the initial count through the culture system employing the 2D organoid.

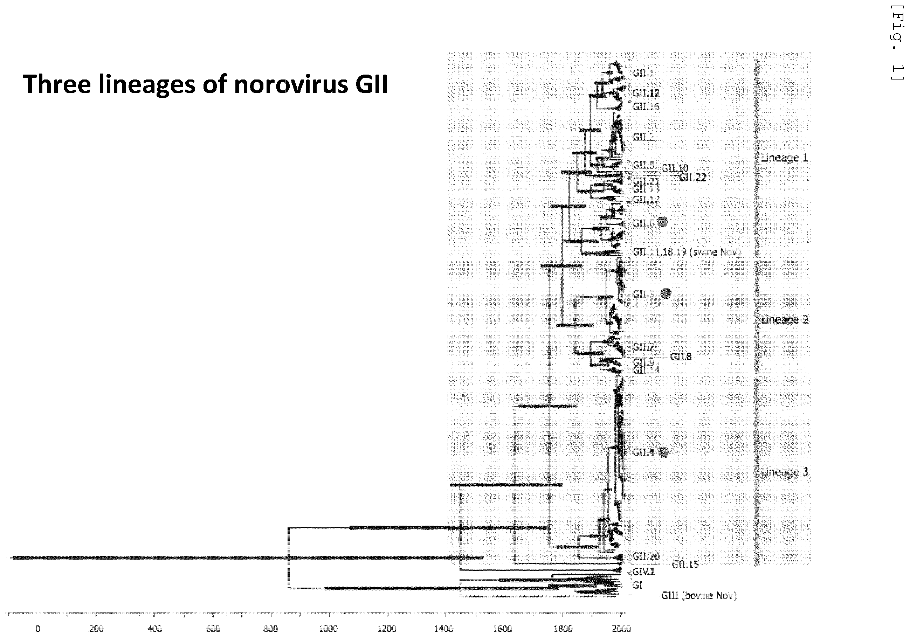

In the Example, there were tested strains of human norovirus obtained from feces of human norovirus infection patients through the aforementioned procedure and stocked before the test. Specifically, three strains belonging to the genetic group 2 (GII) which accounts for 80 to 90% of the infected patients were selected as test targets. The isolated strain lineages included in GII (FIG. 1) were GII.6 belonging to "Lineage 1," GII.3 belonging to "Lineage 2," and GII.4 belonging to "Lineage 3" (GII.6: K2017 strain, GII.3: TCH2004 strain, and GII.4: K2017 strain).

### (2) Virus-like particles (VLPs)

A VLP (virus-like particle) is a particle extremely similar to a human norovirus and obtained by incorporating a structural protein domain of the human norovirus genome into a baculovirus and expressing the genetic domain in insect cells. Since the structure of VLP is completely the same as that of a particle of human norovirus, VLP has the same immunogenicity as that of the virus particle. However, VLP having no genomic RNA therein (i.e., conceptually hollow) is not infectious.

In the Example, a nucleotide sequence (AB447456) encoding a structural protein domain of norovirus GII.4 was incorporated into a baculovirus, and the sequence was expressed in insect cells (SF9 cells). The expression product was used in the Example.

### (3) Agents

In the Example, (3S,4R,5R,6S)-3-fluoro-6-methyltetrahydro-2H-pyran-2,4,5-triyl triacetate (product of Merck, and hereinafter may be referred to as 2fp-fucose) was used as a fucose analog. 2fp-Fucose is one embodiment of the fucose analog represented by formula (III), wherein each of R¹, R³, and R⁴ is -OAc, and R² is F, and more specifically, a fucose analog represented by the following formula (V).

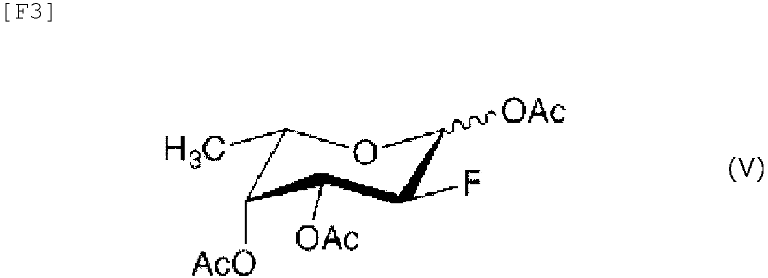

In the Example, 1,2,3,4-tetra-O-acetyl-6,7-dideoxy-L-galacto-hept-6-ynopyranose) (product of Peptide Institute, and hereinafter may be referred to as 6a-fucose) was used as another fucose analog. 6a-Fucose is one embodiment of the fucose analog represented by formula (III), wherein each of R¹, R², R³, and R⁴ is -OAc, and R⁵ is -C=CH, and more specifically, a fucose analog represented by the following formula (VI).

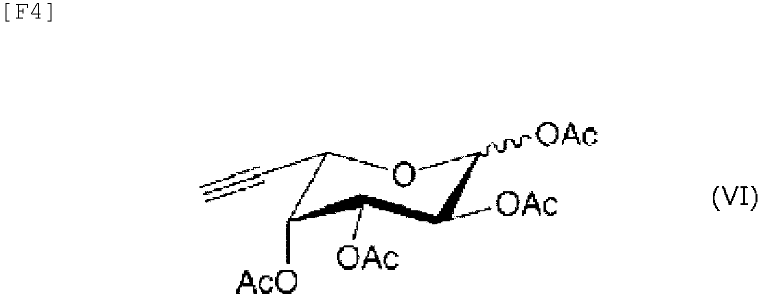

As comparative compounds, commercial antiviral agents; i.e., Sofosbuvir, Lamivudine, Ribavirin, and Mizoribine were provided.

### [Human norovirus proliferation inhibition test by use of 2fp-fucose]

### (1) Cytotoxicity of 2fp-fucose to 2D organoid

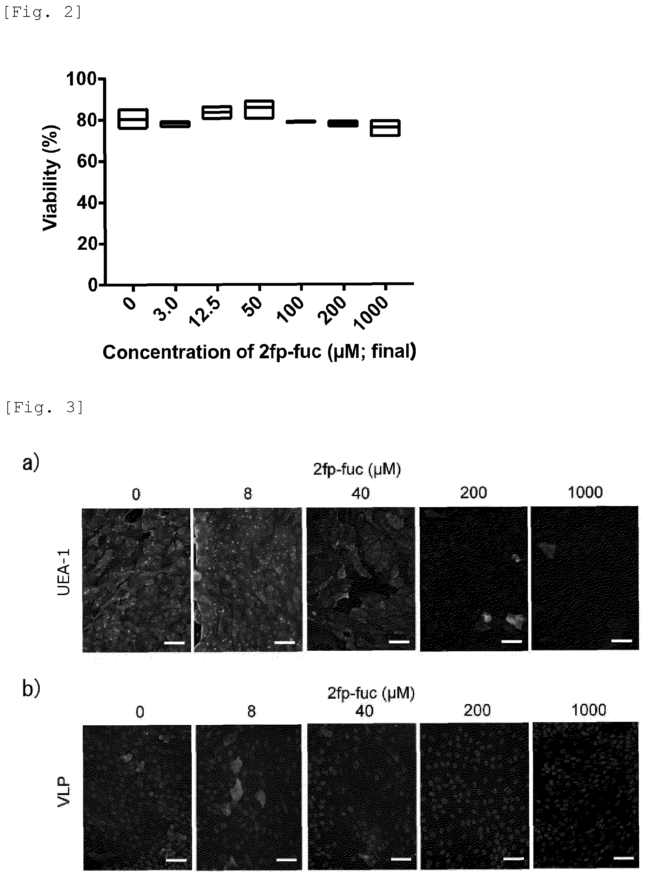

The 2D organoid was treated with 2fp-fucose (3.0 to 1,000 µM) for 7 continuous days. In each case, the number of dead cells was determined through Trypan blue staining. The test was repeatedly performed three times. FIG. 2 shows the results. In FIG. 2, an error bar represents an SEM.

As shown in FIG. 2, no significant difference was observed in cell viability between 2fp-fucose treatment (all concentrations) and no treatment (0 **µ**M) 7 days after the start of treatment.

Thus, the safety of 2fp-fucose to cells has been confirmed by the above results.

### (2) Effect of 2fp-fucose treatment on HBGA expression on the surface of 2D organoid

The 2D organoid was subjected to a 2fp-fucose treatment (0, 8, 40, 200, and 1,000 **µ**M) for 4 continuous days. Reduction of fucose-modified proteins including HBGA formed on the cell surfaces was investigated by checking binding of FITC (phosphor)-labeled UEA-1 (ulex europaeus agglutinin-1). Notably, UEA-1 reacts with a fucose-modified protein.

Through the 2fp-fucose treatment, the amount of **α-**link fucose including HBGA formed via gene expression on the surface of the 2D organoid was found to decrease in a concentration-dependent manner with respect to 2fp-fucose. The tendency was found to be more obvious, particularly when the concentration exceeded 200 **µ**M (FIG. 3(a)). FIG. 3(a) includes monochrome photographic images.

Separately, VLPs of norovirus GII.4 were labeled with AF488 (Alexa Fluor 488; green phosphor) and purified. The thus-prepared labeled VLPs were added to the 2fp-fucose-treated 2D organoid, and a state of binding of VLPs to the surface of the 2fp-fucose-treated 2D organoid was observed. Similar to the case of UEA-1, the 2fp-fucose concentration in the treatment was 0, 8, 40, 200, and 1,000 **µ**M (for 4 treatment days). As a result, the VLP binding amount was found to decrease in a concentration-dependent manner with respect to 2fp-fucose. When the 2fp-fucose concentration exceeded 40 **µ**M, definite reduction in VLP adsorption on the surface of 2D organoid was observed (FIG. 3(b)). FIG. 3(b) includes monochrome photographic images.

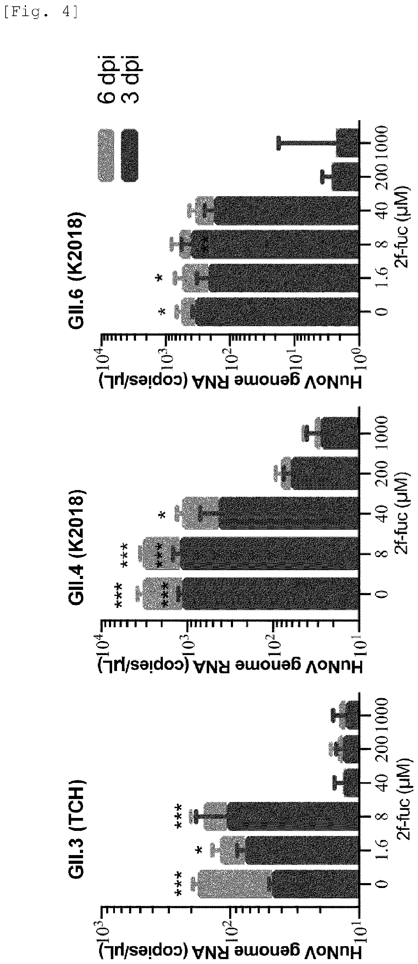

The 2fp-fucose concentration at which obvious reduction in adsorption of the VLP (GII.4) onto the surface of the 2D organoid was observed was "40 **µ**M." The concentration is lower than "200 **µ**M or higher" (mentioned hereinbelow) at which the effect of inhibiting proliferation of isolated norovirus GII.4 was clearly confirmed as shown in FIG. 4. In contrast, a clear decrease in fluorescence intensity of the UEA-1 attributed to binding to 2D organoid occurred at a 2fp-fucose concentration "higher than 200 **µ**M." This concentration was the same as the 2fp-fucose concentration at which the substantial effect of inhibiting proliferation of isolated norovirus GII.4 was clearly confirmed as shown in FIG. 4. In other words, conceivably, when the 2fp-fucose concentration was 40 **µ**M, the action of an unknown human norovirus receptor (or receptors) present on the surface of the 2D organoid was weakened, but was not completely impaired. When VLPs are trapped by a structure which can adsorb human norovirus particles and which is present on the surface of the 2D organoid, fluorescence occurs. However, a considerable number of untrapped VLPs are conceivably incorporated into the 2D organoid. The VLPs which have been incorporated into the 2D organoid are rapidly decomposed, and the fluorescent dye disappears. The phenomenon can be recognized by a reduction in fluorescent intensity.

Meanwhile, UEA-1 can bind to a wide variety of fucose-modified proteins. When the 2fp-fucose concentration exceeds 40 **µ**M and reaches about 200 **µ**M, 2fp-fucose is widely spread over the proteins to be modified with fucose, the proteins being present in the cells and on the surfaces of the cells. The presence of 2fp-fucose in such a state is thought to achieve a substantial level of inhibition of binding UEA-1 on the cell surfaces. There are a variety of fucose-modified proteins in the cells, and the modified proteins should be involved in reaction after incorporation of human norovirus particles. The actions of fucose-modified proteins in the cells are impeded via inhibition of formation of the proteins by 2fp-fucose at a concentration elevated to about 200 µM. As a result, intracellular proliferation of human norovirus is conceivably inhibited.

As described above, highly provably, the fucose analog serving as the active ingredient of the anti-norovirus agent of the present invention prevents incorporation of human norovirus into cells and inhibits proliferation thereof in the cells.

### (3) Effect of 2fp-fucose treatment on proliferation of human norovirus

The effect of 2fp-fucose treatment on proliferation of human norovirus was investigated for each lineage of norovirus. Specifically, after the 2fp-fucose treatment for 3 days, the 2D organoid was infected with each of the aforementioned norovirus strains GII.6, GII.3, and GII.4, and the viral proliferation amount was determined on days 3 and 6 after infection (FIG. 4). As a result, as shown in FIG. 4, GII.3 strain was found to exhibit complete viral proliferation inhibition at 40 **µ**M or higher; GII.6 strain was found to exhibit complete viral proliferation inhibition at 200 **µ**M or higher; and GII.4 strain was found to exhibit slight viral proliferation inhibition at 40 **µ**M and complete viral proliferation inhibition at 200 **µ**M or higher. The test results have proven that these lineages of human norovirus exhibit complete viral proliferation inhibition at 200 **µ**M or higher.

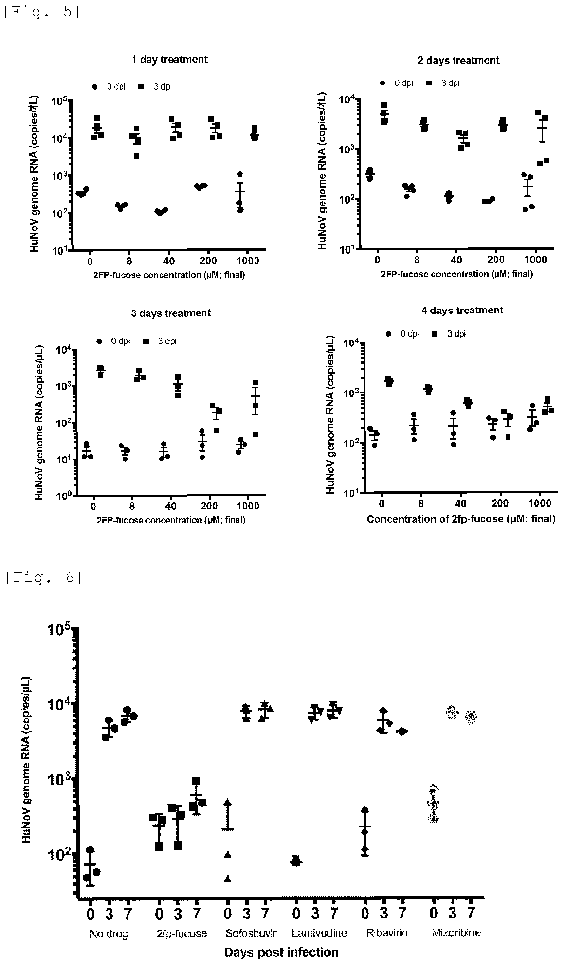

Next, there was investigated the effect of the period of time (the number of days) of the 2fp-fucose treatment performed in advance on the anti-human norovirus action of the fucose analog. Specifically, the strain GII.4 was treated with 2fp-fucose (several concentrations) for 1 to 4 days. FIG. 5 shows the results. As shown in FIG. 5, in the groups of 1-day treatment and 2-day treatment, human norovirus was found to **≥**100-fold proliferate 3 days after inoculation, indicating no proliferation inhibition effect by 2fp-fucose or concentration dependency of the inhibitory effect. However, in the group of 3-day treatment, the proliferation inhibition effect was observed by the 200 µM treatment with a certain range of variation in effectiveness. In the group of 4-day treatment, the proliferation inhibition effect was initially observed by 8 **µ**M treatment, and the effect increased in a concentration-dependent manner to a 2fp-fucose concentration of 200 **µ**M. In contrast, in the case of 1,000 **µ**M treatment, slight proliferation of human norovirus was observed. Thus, the highest proliferation inhibition effect was obtained in the 2D organoid system through a 200 **µ**M treatment for 3 to 4 days. The test was repeatedly performed three times. Error bars denote SEMs.

### (4) Comparison in effect between 2fp-fucose and other viral proliferation inhibitors

The 2D organoid was treated with an agent for 3 days. The tested agents were (a) 2fp-fucose (200 **µ**M), (b) Sofosbuvir (1 **µ**M) , (c) Lamivudine (1 **µ**M), (d) Ribavirin (400 **µ**M), and (e) Mizoribine (100 **µ**M). Subsequently, the thus-treated 2D organoid was infected with human norovirus (the norovirus strain GII.4), and the proliferation inhibition effect was determined (FIG. 6). The test was repeatedly performed three times. Error bars denote SEMs. Only in the case of 2fp-fucose, no substantial increase in viral RNA titer was observed from day 0 to day 7, indicating the effect of inhibiting proliferation of human norovirus. In all cases of the other inhibitors, viral RNA titer rose to a level almost the same as that observed in the agent-free group (No drug), indicating no effect of inhibiting proliferation of human norovirus.

### [Studies on mechanism of human norovirus proliferation_ inhibition by 2fp-fucose]

### (1) FUT gene expression profile and cytotoxicity by 2fp-fucose

After incorporation into cells, 2fp-fucose is involved in **α-**fucose modification by the mediation of fucosyltransferase (fucose transferase) (FUT). However, since 2fp-fucose differs from fucose in terms of a part of side chains, further sugar chain extension from 2fp-fucose is inhibited. Therefore, 2fp-fucose is a fucose analog which can inhibit complete glycosylation by virtue of the mechanism. 2fp-Fucose inhibits glycosylation by all types of fucosyltransferase which have ever been found in mammalian cells, specifically including FUT1 and FUT2 involved in **α**1-2 fucosylation and FUT3-11 involved in **α**1-3 fucosylation.

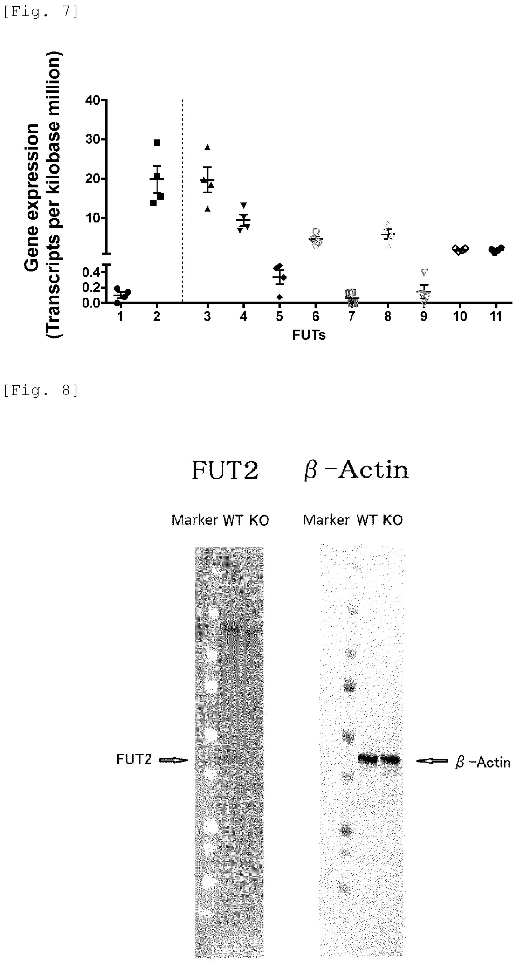

In this experiment, the level of formation of FUT (including FUT1 to FUT11) via gene expression in the human norovirus-susceptible 2D organoid was investigated through RT-PCR. FIG. 7 shows the results.

As shown in FIG. 7, among FUT1 and FUT2, involved in **α**1-2 fucosylation, FUT2 was actively formed through gene expression, but the amount of FUT1 formed through gene expression was about 1/200 that of FUT2. Among FUT3 to FUT11 members, which are involved in **α**1-3 fucosylation, formation of FUT3, FUT4, FUT6, FUT8, FUT10, and FUT11 via gene expression was confirmed. However, formation FUT5, FUT7, and FUT9 vis gene expression was confirmed merely at a background level. Thus, although the functions of FUT2, FUT3, FUT4, FUT6, FUT8, FUT10, and FUT11, which are intrinsically formed through gene expression in the 2D organoid, were impeded by 2fp-fucose, no effect on cell viability was confirmed.

### (2) Studies on relationship between proliferation of human norovirus and HBGA by use of FUT2 gene-knocked-out cells

The relationship between HBGA secretor status and expression of an FUT2 gene in the 2D organoid was investigated. Specifically, the FUT2 gene of the 2D organoid was knocked out, and expression of the FUT2 gene in the knocked-out 2D organoid was confirmed through western blotting (FIG. 8). In FIG. 8, "WT" denotes a wild-type 2D organoid as a control, and "KO" denotes a knocked-out 2D organoid thereof. As a marker, "Bio-Rad Precision Plus Protein™ Bicolor Standard 1610374" was used. The left electrophoretogram shows the test results of FUT2, and the right electrophoretogram show those of **β**-actin (as control). In the left electrophoretogram, a band was observed only in the WT lane locating between the 5th and the 6th size markers and corresponding to FUT2. In the right electrophoretogram, bands were observed in the WT lane and the KO lane locating between the 5th and the 6th size markers and corresponding to **β-**actin. Thus, the amount of FUT2 formed via gene expression in the knocked-out 2D organoid was reduced to a level which cannot be detected through western blotting. Since the amount of **β-**actin was equivalent in the non-knocked-out 2D organoid (wild type) and in the knocked-out 2D organoid, the amounts of cells supplied to electrophoresis and those to western blotting were proven to be the same.

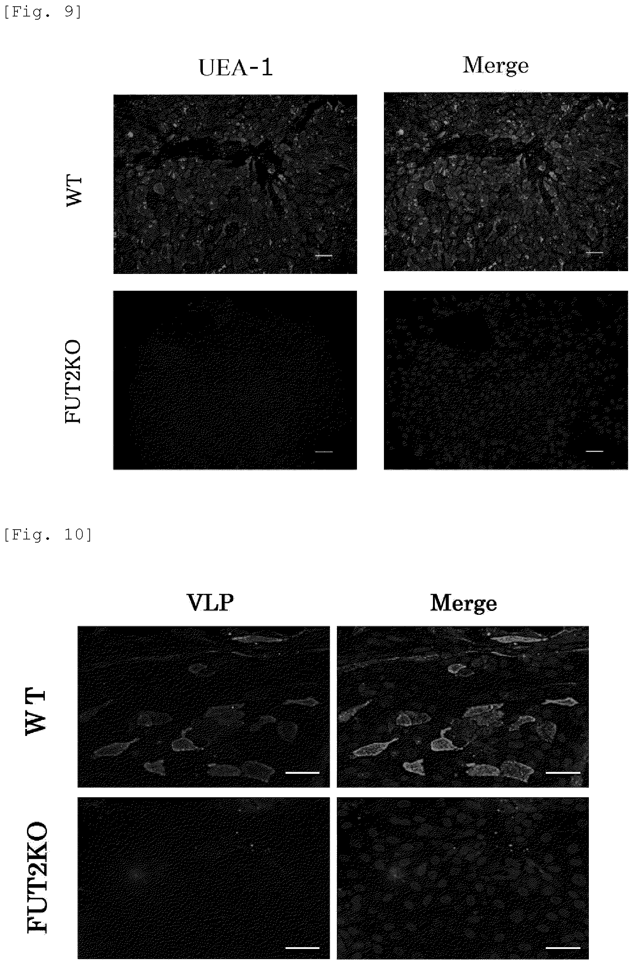

FIG. 9 shows the results of studies on the state of HBGA on the cell surfaces in an FUT2 gene-knocked out 2D organoid and in the wild-type 2D organoid, on the basis of binding of FITC (phosphor)-labeled UEA-1 as an index. In FIG. 9, photographs of "UEA-1" are monochrome photo-images showing the distribution and intensity of fluorescence when FITClabelled UEA-1 was brought into contact with WT or FUT2KO. Photographs of "Merge" are composites of the "UEA-1" images and those obtained by nuclear staining with Hoechst (registered trademark) 33342 (blue). As shown in FIG. 9, when formation of FUT2 via gene expression was inhibited in a pin-point manner through knocking out the FUT gene, no substantial HBGA was observed on the surface of 2D organoid.

Next, green phosphor AF488-labelled VLPs of GII.4 were brought into contact with a human norovirus-knocked-out 2D organoid or the wild-type 2D organoid, and binding of VLPs to the surface of each 2D organoid was confirmed (FIG. 10). As a result, no substantial binding of VLPs to the knocked-out 2D organoid was observed. In the monochrome photo images of FIG. 10, photographs of "Merge" are composites of the "VLP" images and those obtained by nuclear staining with Hoechst (registered trademark) 33342 (blue).

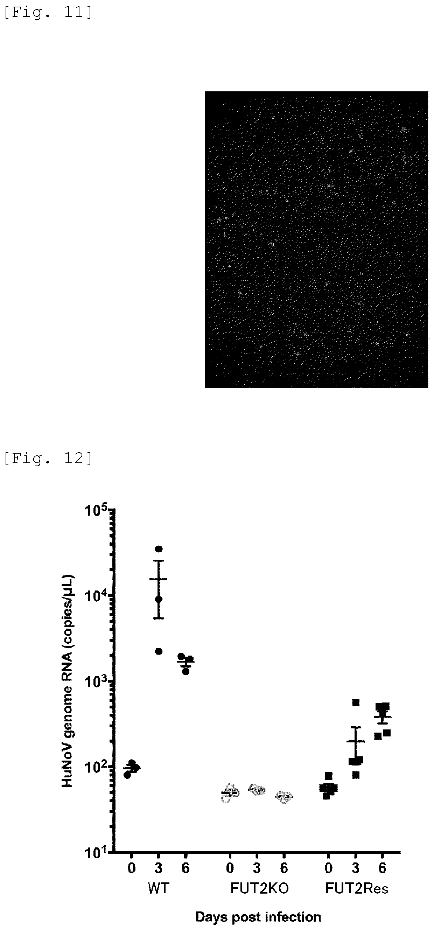

Next, the FUT2 gene was returned to the above FUT2 gene-knocked-out 2D organoid (i.e., a rescue operation) for trying to form FUT2 again via gene expression. The 2D organoid infected with human norovirus was red-stained with an anti-human norovirus VP1 antibody. As a result, only a limited amount of stained cells were observed among the cells spread in the vision field. The presence of the stained cells indicated the presence of a small amount of human norovirus-infected 2D organoid onto which VLPs were attached (bright spots in the monochrome photo image in FIG. 11). The image suggested that HBGA was formed again on the surface of the knocked-out 2D organoid at a low efficiency.

Finally, each of the wild-type 2D organoid (WT), the FUT2 gene-knocked-out 2D organoid (FUT2KO), and the FUT2 gene-returned 2D organoid (FUT2Res) was infected with human norovirus (the aforementioned norovirus GII.4 strain). Percent 2D organoid proliferation was determined in each case, and the results were compared (FIG. 12).

As shown in FIG. 12, in the case of WT, a rise in RNA titer was observed on day 3 and day 6, confirming proliferation of human norovirus. However, in the case of the FUT2 gene-knocked-out 2D organoid, no rise in RNA titer was observed, indicating that no proliferation of human norovirus occurred. In the knocked-out 2D organoid cells to which the FUT2 gene had been returned, a rise in RNA titer was observed, although the rising rate was lower than that in the case of the wild type (WT), confirming that human norovirus is in ready for proliferation.

FUT2Res cells are prepared by incorporating the FUT2 gene to FUT2KO cells. Thus, FUT2 gRNA and Cas9, working for knocking out FUT2, are continuously expressed in the cells. As a result, a newly incorporated FUT2 gene is gradually knocked out. Conceivably, even when the FUT2 gene is newly incorporated into FUT2KO cells, the human norovirus proliferation performance cannot be revived to such a level as attained in the case of the wild type, and thus the percent proliferation is inferior to the case of WT.

The organoid gains human norovirus infection susceptibility in the process of differentiation to intestinal cells. The above test results match the hypothesis that FUT2 works in the differentiation process and directly or indirectly affects maturation of a human norovirus infection receptor that takes human norovirus into cells at the cell surfaces. However, as described above, there has been elucidated no precise mechanism including a course of formation of FUT2 via gene expression to generation of infection-susceptible cells.

### [Human norovirus proliferation inhibition test by use of 6-alkynylfucose]

To a medium containing the 2D organoid, 6a-fucose was added so as to adjust a final concentration of 0 **µ**M, 1.6 **µ**M, 8 **µ**M, 40 **µ**M, and 200 **µ**M, and culturing for differentiation induction was performed for 4 days. The culturing was performed by means of a 96-well plate in which 3 wells were dedicated for each 6a-fucose addition system, and one test plate was used for one human norovirus tested. The human norovirus tested included the GII.4 strain and GII.6 strain. The culturing was performed in a CO₂ gas incubator controlled at 37°C.

On day 4 of culturing, the medium was removed, and the plate was washed with a new medium. Subsequently, each human norovirus was added at 10⁵ RNA copies to the plate for infection, and static culturing was performed for 3 hours. After completion of the infection treatment, human norovirus remaining in the medium was removed together with the medium, and the plate was washed thrice with a new medium.

After washing, 6a-fucose was added again to the new medium so as to adjust a final concentration to 0 **µ**M, 1.6 **µ**M, 8 **µ**M, 40 **µ**M, and 200 **µ**M, and culturing was performed for 6 days.

A portion (10 **µ**L) of the culture medium was collected from each well at three timings: (a) immediately after the addition to the new medium, (b) on day 3, and (c) on day 6. The sample was centrifuged at 10,000g for 10 minutes, and the cell precipitate was removed. The human norovirus genomic RNA contained in the supernatant (1 **µ**L) was quantitated through real-time RT-PCR.

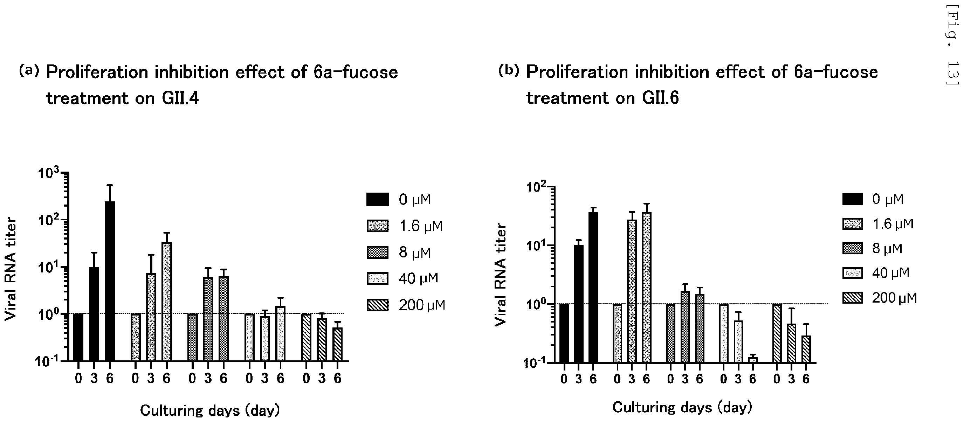

FIG. 13 shows the results. In FIG. 13, the effects of use of 6a-fucose are shown with respect to (a) human norovirus GII.4 strain and (b) human norovirus GII.6 strain. The vertical axis in FIG. 13 represents the copy number of human norovirus genomic RNA (RNA titer), and the numeral under each bar graph represents the time of culturing from the start of the test.

FIG. 13(a) shows the proliferation inhibition effect on the GII.4 strain. The effect emerged at a 6a-fucose concentration of 1.6 **µ**M. In the untreated group, a **≥**100-fold rise (with respect to start of culturing) in RNA titer was observed on culture day 6. In contrast, RNA titer was suppressed to **≤**100 times the titer at the start of culturing in each 6a-fucose treatment group (any concentration). More specifically, when the 6a-fucose concentration was 8 **µ**M, the rise in RNA titer was further suppressed as compared with the case of 1.6 **µ**M, and the rise was merely about 10 times the titer at the start of culturing. When the 6a-fucose concentration was 40 **µ**M, the titer was slightly higher on day 6 than the initial value at the start of culturing, but no substantial increase in RNA titer was observed. When the 6a-fucose concentration was 200 **µ**M, the rise in RNA titer was completely inhibited.

FIG. 13(b) shows the proliferation inhibition effect on the GII.6 strain. The effect was not recognized at a 6a-fucose concentration of 1.6 **µ**M. However, human norovirus genome proliferation was actively suppressed at 8 **µ**M, and a slightly higher proliferation level than that of day 0 was observed on culture days 3 and 6. When the 6a-fucose concentration was 40 **µ**M, the proliferation was completely inhibited.

Notably, cultured cells (2D organoid) in the wells other than the wells in which proliferation was observed in culturing were still alive in the form of mono-layer sheet. Thus, no cytotoxicity attributed to addition of 6a-fucose was observed in any of the tested concentrations.

### [Studies on the effect of treatment with 2fp-fucose or 6a-fucose after viral infection]

### (1) About test system

After differentiation induction, about 4 days are required for complete differentiation from the organoid to the small intestine epithelial cells. Then, cell division is substantially terminated, and, if no medium replacement is conducted, cell death starts on day 7 to day 9. As is also clear from the test results, the effect of a fucose analog emerges on day 2 of addition, and the effect peaks on day 4. Meanwhile, human norovirus increases in the culture medium (i.e., release of new virus to the culture medium begins) on the day following the infection, and proliferation can be observed. The proliferation lasts until day 6.

Generally, in the human body, the primary infection of small intestine epithelial cells with human norovirus and viral proliferation in the cells cause the death of small intestine epithelial cells. When the dead cells exfoliate, stem cells present in the intestinal crypt undergo cell division, and normally differentiation-induced epithelial cells are supplied. Shortly, intestinal villus is regenerated. The thus-supplied epithelial cells are newly infected with human norovirus released from the small intestine epithelial cells to the intestinal tract through the primary infection/intracellular proliferation. When new human norovirus infection occurs, the second cycle of proliferation of human norovirus starts in the newly supplied cells. At this stage, viral proliferation control by immunoresponse of the host generally begins. Thus, conceivably, viral infection/proliferation is gradually suppressed.

In order to design an effective administration form of the anti-norovirus agent of the present invention adapted to the aforementioned human norovirus infection/proliferation cycle, how the fucose analog acts on the human norovirus infection/proliferation cycle is desirably monitored over time.

However, currently, no laboratory animal is available for monitoring the human norovirus infection/proliferation cycle. Thus, difficulty is encountered in integrally monitoring proliferation of human norovirus, cell regeneration, new cell supply, re-infection, and immunoresponse in vivo.

Under such circumstances, the time-over effect of administration of a fucose analog after infection with human norovirus was determined in vitro under the following conditions.

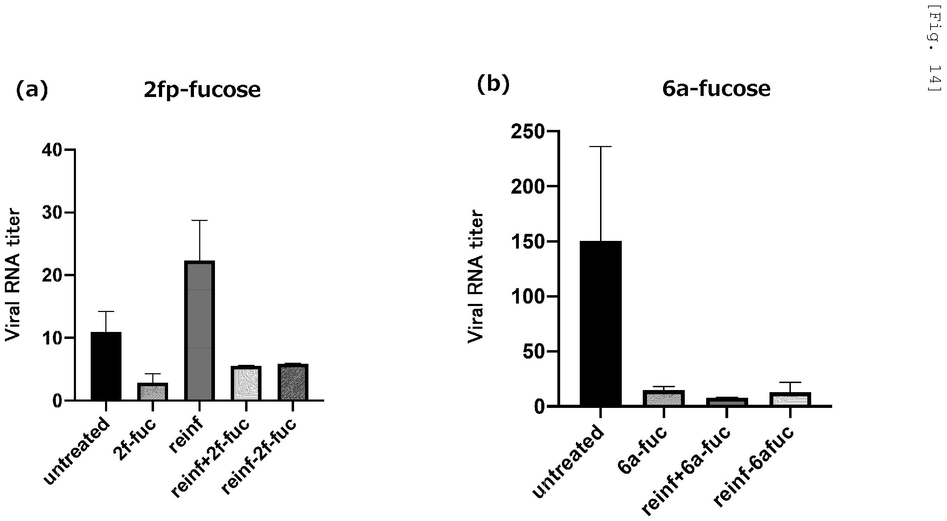

### (2) Test system and test results (FIG. 14)

(a) The 2D organoid was disseminated to a 96-well plate. After completion of differentiation induction, two fucose analogs (i.e., 2fp-fucose and 6a-fucose) were each added on day 5 to a concentration of 200 **µ**M, to thereby provide addition groups. The results are shown in FIG. 14(a) and FIG. 14(b), respectively. A non-addition group (untreated group) was also provided. Subsequently, the medium was removed from the wells on day (5 + 2), and the plate was washed. Then, infection with the GII.4 strain or the GII.6 strain was performed.

After the infection treatment, the human norovirus remaining in the medium was removed together with the medium, and the plate was washed thrice with a new medium. After washing, the aforementioned active ingredients were each added to the new medium to a concentration of 200 **µ**M (addition groups), and no addition was performed with respect to the untreated (i.e., non-addition) group. Culturing was performed for 3 days. A portion (10 **µ**L) of the medium was sampled from each well, and the samples were centrifuged at 10,000g for 10 minutes, to thereby remove cell precipitates. The amount of human norovirus genomic RNA in the supernatant (1 **µ**L) was determined through real-time RT-PCR.

At this stage, the untreated group exhibited a **≥**10-fold rise in RNA titer with respect to the 2fp-fucose administration system and a **≥**150-fold rise in RNA titer with respect to the 6a-fucose administration system. In contrast, no rise in RNA titer (i.e., proliferation of human norovirus) was observed in the two addition groups. The results are shown in FIGs. 14(a) and 14(b). In the graphs, the two bars on the left side correspond to the untreated group and the 2f-fuc- or 6a-fuc-addition group.

(b) In the untreated group, human norovirus infection-susceptible cells still remained. Thus, new infection of the remaining infection-susceptible cells with human norovirus may possibly occur. Accordingly, the effect of the anti-norovirus agent of the present invention on the remaining infection-susceptible cells was investigated through the following procedure.

In a manner similar to that of the (a) described above, the untreated group was subjected to culturing for 3 days, and then the medium of each culture was removed. The system was washed and infected again with human norovirus (i.e., reinfection: reinf.), and the virus was removed together with the medium. After completion of washing, a new medium was added, and culturing was performed for 3 days. In a manner similar to the above, the amount of human norovirus released to the supernatant was measured as an RNA titer. In the reinfection test, a **≥**20-fold rise in RNA titer with respect to the initial infection observed in the aforementioned (a) was observed (see the bars of reinf in FIG. 14(a)). That is, the cells were newly infected with the norovirus via the re-infection process, and the virus concentration of the medium was found to increase as compared with the case of initial (primary) infection.

(c) Next, the effect of the fucose analog on re-infection was investigated. Generally, the onset of norovirus symptoms in humans occurs about 24 to about 48 hours after infection with human norovirus. Therefore, there was assumed a model case of administration of an effective amount of the anti-norovirus agent of the present invention immediately after the onset. In this case, according to the aforementioned findings, the anti-viral effect was expected to be attained on the second day or later after the initial infection with human norovirus. Also, in the presence of the fucose analog, 3 days after the fucose analog treatment, cells derived through differentiation induction of the crypt stem cells after the initial infection were to be brought into contact with the virus.

In the test system, the effect of the anti-norovirus agent of the present invention provided after human norovirus infection was estimated on the basis of the following study. Specifically, among the fucose analog-treated 2D organoid cells, the living cells on growth day 3 (i.e., most of them were not infected with human norovirus, by virtue of the effect of the fucose analog) were subjected to a secondary infection operation (i.e., reinfection). The effect of the fucose analog on the reinfection was investigated.

In a specific procedure, the addition groups were prepared through the same procedure as employed in (a) above. The primarily infected cells were cultured in the presence of the fucose analog (200 µM) for 3 days, and the medium was removed from each culture system. After completion of washing, the cells were infected again with human norovirus, and then the virus was removed together with the medium. After completion of the next washing, a medium containing the fucose analog (200 µM) (represented by Reinf+2f-fuc or Reinf+6a-fuc), or a fucose analog-free medium (represented by Reinf-2f-fuc or Reinf-6a-fuc) was added thereto, and culturing was performed for further 3 days. Similar to the above case, the amount of human norovirus released to the supernatant was determined as an RNA titer. The results are shown in FIGs. 14(a) and 14(b). In the graphs, the two bars on the right side correspond to (Reinf+2f-fuc and Reinf+6a-fuc) or (Reinf-2f-fuc and Reinf-6a-fuc).

As is clear from FIG. 14, the cells primarily infected human norovirus and grown for 3 days in the presence of the active ingredient were resistive to reinfection with human norovirus, regardless of addition of the fucose analog after reinfection.

### (3) Conclusion

The test results described above have revealed the following. That is, when a human subject is primarily infected with human norovirus, and the subject continuously takes an effective amount of the anti-norovirus agent of the present invention from the onset, continuous emergence of symptoms attributed to reinfection possibly occurring after day 2 or day 3 of the primary infection can be effectively suppressed, although it is difficult to avoid all the symptoms caused by primary infection. Thus, use of the anti-norovirus agent of the present invention is remarkably advantageous for mitigating the gravity and the period of pain of human norovirus infection patients. Furthermore, in the case where the anti-norovirus agent of the present invention is used to a human norovirus patient after onset of symptoms, the period of human norovirus excretion to feces, which is a problem as a cause of spread of human norovirus infection, is expected to be considerably shortened.

## Claims

1. An anti-human norovirus agent comprising, as an active ingredient, a fucose analog which inhibits glycosylation by fucosyltransferase (FUT) or a salt thereof.

2. The anti-human norovirus agent according to claim 1, wherein the fucosyltransferase, inhibited from being glycosylated by the fucose analog, includes FUT2.

3. An anti-human norovirus agent comprising, as an active ingredient, a fucose analog represented by formula (III) or (IV): 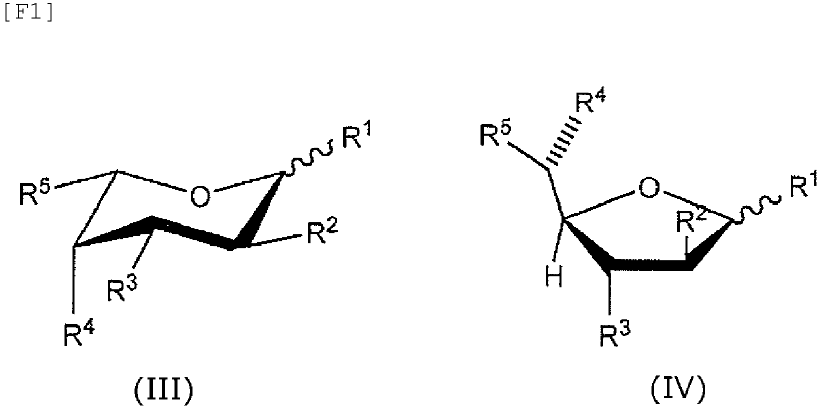 [wherein each of the formula (III) and the formula (IV) represents an α-anomer or a β-anomer;
each of R¹, R³, and R⁴ is -OH or -OAc;
R² is a halogen atom which is F (fluorine atom) or Cl (chlorine atom), -OH, or -OAc; and
R⁵ is -CH₃, -C=CH, -C≡CCH₃, or -CH₂C≡CH.] or a salt thereof.

4. The anti-human norovirus agent according to claim 3, wherein, when R² is the halogen atom, at least one group of R¹, R³, and R⁴ is -OAc; and when R⁵ is -C=CH, -C≡CCH₃, or - CH₂C≡CH, at least one group of R¹, R², R³, and R⁴ is -OAc.

5. The anti-human norovirus agent according to claim 3 or 4, wherein, when R² is the halogen atom, R⁵ is -CH₃; and when R⁵ is -C=CH, -C≡CCH₃, or -CH₂C≡CH, R² is -OH or -OAc.

6. The anti-human norovirus agent according to any one of claims 3 to 5, wherein, when R² is the halogen atom, all of R¹, R³, and R⁴ are -OAc; and
when R⁵ is -C=CH, -C≡CCH₃, or -CH₂C≡CH, all of R¹, R², R³, and R⁴ are -OAc.

7. The anti-human norovirus agent according to any one of claims 3 to 6, wherein R² is F (fluorine atom).

8. The anti-human norovirus agent according to any one of claims 3 to 7, wherein R⁵ is -C=CH.

9. The anti-human norovirus agent according to any one of claims 3 to 8, wherein the fucose analog or a salt thereof serving as the active ingredient is a fucose analog represented by formula (III) or a salt thereof.
